# FASCICULE DE BREVET EUROPEEN

(11) **EP 1 502 574 B1**
(45) Date de publication et mention de la délivrance du brevet: **03.11.2010**
(21) Numéro de dépôt: 04300477.9
(22) Date de dépôt: 26.07.2004
(51) Int. Cl.: A61K 8/04, A61K 8/25, A61K 8/88, A61Q 1/00, A61Q 1/02, A61Q 3/02

(54) **Procédé de traitement cosmétique de la peau par pulvérisation et dispositif pour la mise en oeuvre d'un tel procédé.**
Sprühverfahren zur kosmetischen Behandlung der Haut und Vorrichtung zu diesem Zweck
Skin care treatment by spraying and apparatus therefore

(30) Priorité: 01.08.2003 FR 0309573
(43) Date de publication de la demande: 02.02.2005
(73) Titulaire: L'Oréal, 75008 Paris (FR)
(72) Inventeur: Girier-Dufournier, Franck, 75011 Paris (FR); Liechty, Anne, 75018 Paris (FR)
(74) Mandataire: Le Coupanec, Pascale A.M.P.

(56) Documents cités:
- EP-A- 0 997 134
- EP-A- 1 195 155
- WO-A-02/056846
- US-A- 5 665 368
- US-A1- 2002 172 695
- COSMETICS AN TOILETRIES, vol. 111, juillet 1996 (1996-07), pages 57-61, XP009029669
- A.L.L. Hunting: "Decorative cosmetics, a formulary of cosmetic preparations" 1991, Micelle Press vol. 1,
- NATIONAL STARCH AND CHEMICAL COMPANY: "A Unique Selection of Natural and Synthetic Speciality Polymers for Skin Care Applications" SKIN CARE FORMULARY, vol. 2, 1999,
- "Base de données de produits" MINTeL gnpd Retrieved from the Internet: URL:www.gnpd.com>

## Description

La présente invention concerne le traitement cosmétique de la peau, des muqueuses et/ou des phanères, notamment les matières kératiniques non fibreuses.

Plus particulièrement, l'invention vise à proposer un procédé utile notamment pour masquer au moins partiellement des défauts de la peau.

Il est usuel de chercher à camoufler des imperfections de la peau et en particulier du visage ou du cou à l'aide de fonds de teint.

Les fonds de teint conventionnels apportent généralement beaucoup de couvrance et sont utiles pour matifier le teint et masquer des imperfections de couleur de la peau, telles que des rougeurs, mais pas toujours adaptés à masquer également des défauts de relief tels que les rides.

Pour camoufler les rides et autres défauts de relief, des compositions contenant des charges dites à effet de flou ou « soft-focus » sont utilisées. Ces compositions diminuent la perception du relief de la surface sur laquelle elles sont appliquées.

L'application d'une composition contenant des charges à effet de flou, manuellement ou à l'aide d'un applicateur, sur une surface déjà maquillée avec un fond de teint, conduit, au moins partiellement, à un mélange intime des deux compositions, qui entraîne une diminution de l'efficacité de la composition à effet de flou et nuit à la qualité du résultat.

Il subsiste donc un besoin pour bénéficier d'un procédé de traitement cosmétique de la peau permettant notamment de diminuer la perception des défauts de relief, et permettant également, le cas échéant, de masquer des défauts de couleur et de matifier le teint.

Il existe par ailleurs un besoin pour améliorer encore la qualité du maquillage obtenu au moyen d'une composition contenant au moins une charge à effet de flou.

Il existe encore un besoin pour dissimuler des défauts sans générer d'effet de masque et garder à la surface maquillée un aspect naturel.

L'invention vise à répondre à tout ou partie de ces besoins.

La présente invention a pour objet, selon un premier de ses aspects, un procédé de traitement cosmétique, notamment de maquillage, d'une surface de la peau, des muqueuses ou des ongles, ce procédé comprenant l'application, par pulvérisation sur ladite surface, d'une composition cosmétique, dite à effet de flou, cette composition comportant, dans un milieu physiologiquement acceptable, au moins une charge de taille spécifique et permettant de conférer à la composition un indice de flou H supérieur ou égal à 40 % et un facteur de transmission hémisphérique Th supérieur ou égal à 70 %.

Par « pulvérisation », on désigne une distribution de préférence sous la forme d'un nuage de plus ou moins fines particules, notamment de gouttelettes.

L'application par pulvérisation de la composition peut permettre un dépôt sensiblement uniforme de celle-ci, contribuant à camoufler encore mieux les défauts de relief de la peau.

L'application de la composition à effet de flou peut être précédée par l'application sur la surface à traiter d'une composition de base, notamment colorée, par exemple un fond de teint conventionnel.

Une méthode de maquillage selon l'invention peut donc impliquer, dans une première étape, l'application par tout mode conventionnel à savoir, par exemple, manuel, au moyen d'un applicateur tel qu'un pinceau ou une éponge ou par pulvérisation, d'une composition cosmétique de base, colorée. A cette première couche de maquillage est ensuite superposée, dans une deuxième étape, par pulvérisation, la composition à effet de flou selon l'invention.

La pulvérisation permet avantageusement de déposer sous la forme d'un film de faible épaisseur, par exemple de type « voile », la composition à effet de flou à la surface de la composition de base et de prévenir ainsi tout mélange intime entre les deux compositions qui pourrait être préjudiciable à la manifestation de l'effet de flou recherché.

L'invention peut permettre ainsi d'obtenir un maquillage particulièrement satisfaisant en terme de camouflage, que ce soit des imperfections colorées ou de relief.

L'application de la composition à effet de flou peut encore, en variante, être simultanée à l'application de la composition de base, par exemple grâce à l'utilisation d'un dispositif de pulvérisation comportant au moins deux buses.

La composition à effet de flou peut ou non contenir au moins un agent de coloration, notamment selon que la composition est destinée à être ou non superposée à une composition de base colorée.

Dans le cas notamment où la composition à effet de flou est appliquée directement sur la surface à maquiller et comporte un agent de coloration, la saturation de la composition C* est de préférence comprise entre 17 et 60, mieux entre 20 et 60.

Une valeur de saturation C* supérieure à 17 permet d'obtenir un apport de couleur suffisant tandis qu'une valeur inférieure à 60 permet de ne pas nuire à l'obtention de l'effet de flou et peut permettre également d'obtenir un maquillage naturel.

### Mesure du facteur de transmission hémisphérique Th et calcul de l'indice de flou H

Comme indiqué ci-dessus, la composition cosmétique à effet de flou présente un indice de flou H supérieur ou égal à 40 %, et de préférence supérieur ou égal à 45 %, notamment 50 %, mieux à 60 %, mieux encore supérieur ou égal à 70 %, notamment à 75 %, mieux encore à 80 %.

Le facteur de transmission hémisphérique Th est quant à lui supérieur ou égal à 70 %, notamment supérieur ou égal à 75 %, mieux supérieur ou égal à 80 %, voire à 85 %.

On désigne par « indice de flou H » la grandeur ((Th - Td)/Th).100, dans laquelle Th représente le facteur de transmission hémisphérique et Td le facteur de transmission directe.

Lorsque l'indice de flou H est élevé, la composition procure un effet de flou important, permettant de masquer des défauts en changeant la perception du relief.

Le facteur de transmission hémisphérique Th donne une information sur la transparence de la composition. Plus la valeur Th est élevée, plus la composition est transparente.

Les valeurs Th et Td peuvent être mesurées à l'aide d'un spectrophotomètre et d'une sphère d'intégration, placée derrière la composition que l'on cherche à caractériser.

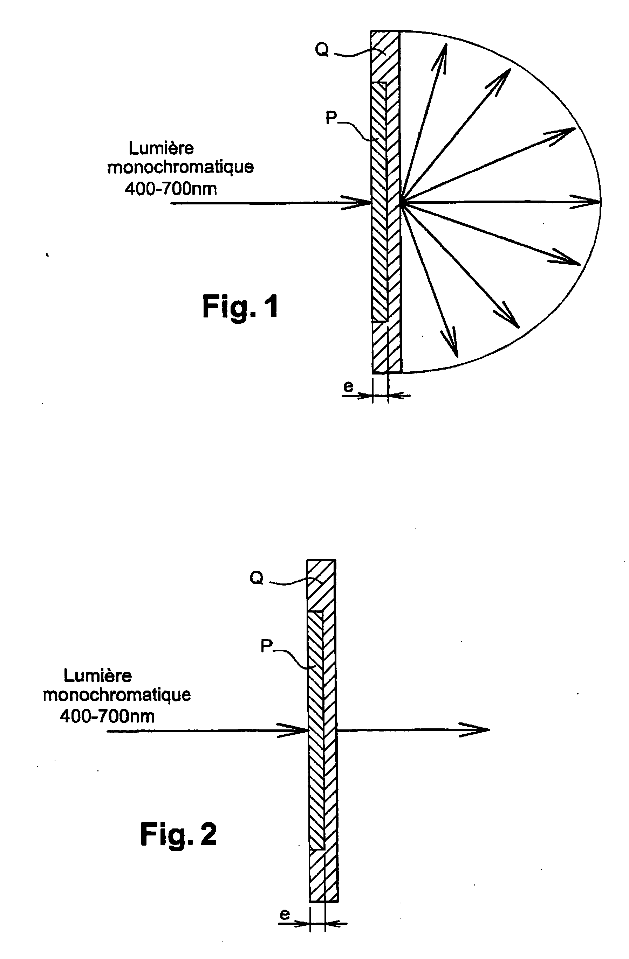

La transmission hémisphérique spectrale Th(λ) de la composition est définie par le rapport entre l'intensité lumineuse de longueur d'onde λ reçue par un échantillon de composition P et l'intensité lumineuse restituée par cet échantillon dans toutes les directions d'un espace délimité par un plan, comme illustré à la figure 1.

La transmission directe spectrale Td(λ) d'un échantillon est définie par le rapport entre l'intensité lumineuse de longueur d'onde λ reçue par l'échantillon et l'intensité lumineuse restituée par cet échantillon dans la même direction de propagation que la lumière incidente, comme illustré à la figure 2.

Les mesures des transmissions spectrales hémisphérique Th(λ) et directe Td(λ) peuvent être effectuées selon le protocole expérimental suivant.

La composition P que l'on cherche à analyser est étalée sur une lame de quartz Q creusée, de manière à former une couche d'une épaisseur e de 20 µm, puis placée dans une étuve pendant 5 minutes à 37°C.

La valeur Th(λ) peut être mesurée à l'aide d'un spectrophotomètre VARIAN Cary 300® et d'une sphère d'intégration de marque Labsphère® placée à l'arrière de la lame de quartz contenant la composition. Le spectrophotomètre est utilisé en mode de transmission diffuse et la longueur d'onde λ de la lumière incidente monochromatique varie de 400 à 700 nm.

La mesure est effectuée en mode transmission % T, à une vitesse de balayage de 240 nm/min et en mode « double reverse ».

Dans un premier temps, il est procédé à un étalonnage en faisant une première mesure sur la lame de quartz Q vide pour obtenir la valeur maximale de l'intensité transmise.

Ensuite, on mesure la transmission hémisphérique spectrale Th(λ) avec la lame de quartz contenant la composition P à analyser.

La valeur Td(λ) est mesurée à l'aide du même spectrophotomètre, utilisé en mode transmission directe et la longueur d'onde λ de la lumière incidente monochromatique incidente varie de 400 à 700 nm. On se place en mode transmission % T, à une vitesse de balayage 240 nm/min, en mode « double ». On place une lame de quartz vide dans un compartiment de référence et la lame de quartz contenant la composition est placée dans un compartiment de mesure, puis Td(λ) est mesuré.

Les facteurs de transmission hémisphérique Th et directe Td sont calculés en établissant respectivement la moyenne de toutes les valeurs spectrales Th(λ) ou Td(λ) obtenues lorsque la longueur d'onde varie de 400 à 700 nm.

### Saturation C*

La saturation C* peut être mesurée dans l'espace colorimétrique CIE L*a*b*C*h, de la façon suivante.

La composition dont on cherche à connaître la saturation est déposée à ras bord d'une coupelle en acier galvanisé et présentant une profondeur de 2,47 mm minimum.

La coupelle est ensuite recouverte d'une lame porte-objet « LMR » de type H1, à bords biseautés, de dimensions 76 x 26 mm, de la société LABO-MODERNE.

La saturation C* est mesurée au moyen d'un spectrocolorimètre CM3700d, le système d'éclairage et d'observation étant en mode réflectance d/8°, les mesures étant effectuées en mode réflexion spéculaire exclue.

L'émission UV est 100 % incluse.

La position zoom est moyenne (MAV), l'ouverture est moyenne, la configuration est CREIMM, l'observation est 10° CIE 1964, l'illuminant est D65.

### Dispositif de pulvérisation

La pulvérisation de la composition à effet de flou peut être effectuée par tous les moyens connus de l'homme du métier, dans la mesure où ceux-ci n'affectent pas les propriétés de flou procurées par la composition.

La pulvérisation peut avoir lieu par exemple grâce à des moyens permettant de pressuriser la composition, par exemple en la soumettant à la pression d'un gaz propulseur.

Ce gaz propulseur peut être conditionné avec la composition ou séparément, celle-ci étant par exemple contenu dans une poche souple à l'intérieur d'un récipient pressurisé.

La composition peut également être pressurisée au moyen d'une pompe, notamment une pompe actionnée manuellement, laquelle peut être à précompression ou non.

La composition peut encore être pulvérisée grâce à un effet Venturi dans un dispositif de pulvérisation de type aérographe.

La composition, quelque soit le mode de pulvérisation adopté, peut être pulvérisée par une ou plusieurs buses de pulvérisation, à canaux tourbillonnaires ou non.

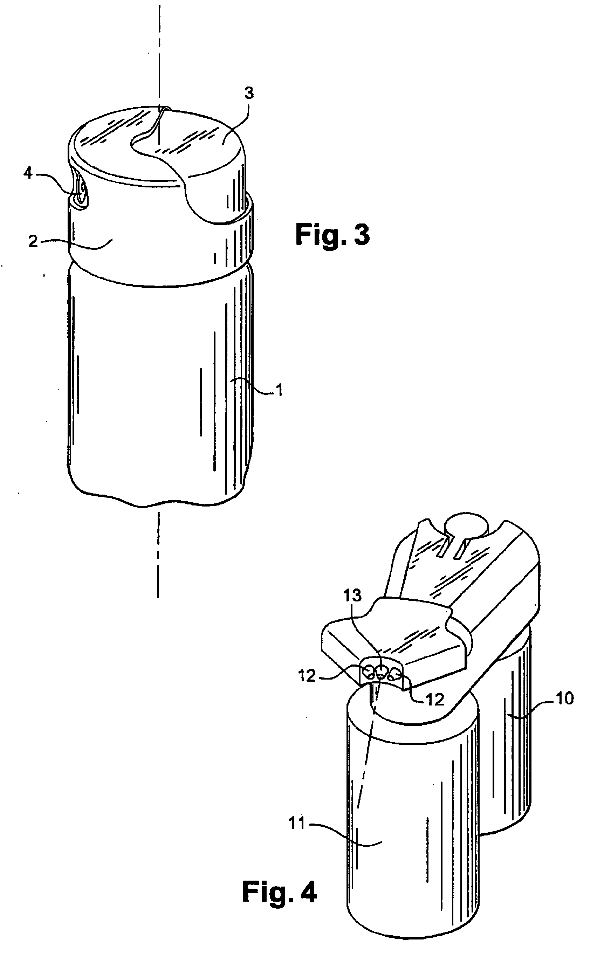

On a représenté aux figures 3 et 4 deux exemples de dispositif de pulvérisation, parmi d'autres.

Le dispositif de la figure 3 est par exemple de type aérosol et comporte un récipient pressurisé 1 contenant la composition et le gaz propulseur, une tête de distribution 2 comportant un bouton-poussoir 3 permettant d'agir sur une valve non apparente et une buse de pulvérisation 4.

On a représenté à la figure 4 un autre exemple de dispositif de pulvérisation, de type aérographe, comportant un premier récipient 10 contenant le gaz vecteur et un deuxième récipient 11 contenant la composition, le gaz vecteur 10 étant émis par exemple par deux buses 12 en avant d'une buse de pulvérisation 13. La dépression qui est créée par l'écoulement du gaz vecteur provoque l'aspiration dans le réservoir 11 de la composition.

Des exemples non limitatifs de dispositifs de pulvérisation sont décrits notamment dans la demande internationale WO 02/47618.

Lorsque la composition est pulvérisée grâce à un gaz propulseur, celui-ci peut être par exemple un gaz comprimé, telle que de l'air ou de l'azote comprimé ou un gaz liquéfiable tel que par exemple le diméthyléther, les alcanes en C₃₋₅, notamment le propane, le n-butane et l'isobutane, le 1,1-difluoroéthane, les mélanges de diméthyléther et d'alcanes en C₃₋₅.

Le rapport pondéral de la composition à effet de flou/gaz propulseur peut notamment aller de 0,05 à 50 par exemple et en particulier de 1,5 à 25.

La pulvérisation peut encore être effectuée au moyen d'un dispositif de pulvérisation sans gaz vecteur ou propulseur, par exemple électromécanique ou thermique.

L'invention a encore pour objet un dispositif de pulvérisation comportant un récipient contenant une composition à effet de flou telle que définie plus haut.

Ce dispositif de pulvérisation peut comporter des moyens pour soumettre la composition à une pression ou une dépression permettant de la pulvériser.

### Charges

Les charges à effet de flou utilisées dans la composition à effet de flou selon l'invention comportent ou sont constituées de particules présentant une taille moyenne en nombre inférieure à 5 µm, par exemple comprise entre 1 µm et 5 µm.

Par « taille moyenne en nombre», on désigne la dimension donnée par la distribution granulométrique statistique à la moitié de la population, dite D50.

Ces particules peuvent être de toutes formes et en particulier être sphériques ou non sphériques.

Les charges à effet de flou selon l'invention peuvent être de toute nature chimique dans la mesure où elles sont compatibles avec une utilisation en cosmétique et où elles n'affectent pas les propriétés attendues de composition.

Elles peuvent ainsi être choisies parmi les poudres de silice et silicates, notamment d'alumine, les poudres de type polyméthyl méthacrylate (PMMA), le talc, les composites silice/TiO₂ ou silice/oxyde de zinc, les poudres de polyéthylène, les poudres d'amidon, les poudres de polyamides, les poudres de copolymères styrène/acrylique, les élastomères de silicone, et leurs mélanges.

Parmi les charges à effet de flou convenant particulièrement bien à l'invention, on peut citer le talc de taille moyenne en nombre inférieure ou égale à 3 microns, par exemple du talc de taille moyenne en nombre de 1,8 micron et notamment celui vendu sous la dénomination commerciale Talc P3® par la société Nippon Talc, la poudre de Nylon® 12, notamment celle vendue sous le dénomination Orgasol 2002 Extra D Nat Cos® par la société Atochem, les particules de silice traitées en surface par une cire minérale 1 à 2 % (nom INCI : hydrated silica (and) paraffin) telles que celles commercialisées par la société Degussa, les microsphères de silice amorphe, telles que celles vendues sous la dénomination Sunsphère par exemple de référence H-53 par la société Asahi Glass, et les micro-billes de silice telles que celles vendues sous la dénomination SB-700® ou SB-150® par la société Miyoshi, cette liste n'étant pas limitative.

La charge à effet de flou aura de préférence un indice de réfraction différent de celui du milieu dans lequel elle se trouve.

La charge à effet de flou peut être sensiblement incolore au sein de la composition.

La charge à effet de flou peut être présente dans la composition cosmétique à effet de flou en une teneur allant de 0,1 à 20 % en poids et notamment allant de 1 % à 12 % en poids par rapport au poids total de la composition, notamment entre 5 et 10 %, par exemple de l'ordre de 8 %.

### Agent de coloration

L'une au moins de la composition à effet de flou et de la composition de base peut comporter au moins un agent de coloration.

La composition à effet de flou selon l'invention peut comporter ou non un agent de coloration.

La composition pourra comporter un agent de coloration lorsqu'elle est destinée à être appliquée directement sur peau nue, par exemple.

Par « agent de coloration », on désigne au sens de la présente invention tout pigment ou colorant ou mélange de pigments et/ou de colorants propre à apporter suffisamment de couleur à la composition pour lui permettre de présenter la saturation C* requise tout en permettant d'obtenir les valeurs d'indice de flou H et de facteur de transmission hémisphérique Th recherchées.

L'agent de coloration peut notamment comporter ou être constitué de particules d'au moins un pigment. Les particules de pigment peuvent avoir subi, le cas échéant, un traitement ayant pour objectif d'augmenter la stabilité de la couleur et faciliter leur incorporation dans la composition. En particulier, des particules de pigment traitées en vu de les rendre hydrophobes seront plus facilement dispersibles dans une phase huileuse, par exemple.

Les particules de pigment peuvent présenter des formes diverses, notamment une forme sensiblement sphérique ou aplatie.

Les particules de pigment peuvent présenter une structure multicouche, et notamment un coeur transparent, par exemple en silice.

De telles particules de pigment à coeur transparent permettent de ne pas opacifier outre mesure la composition, ce qui est favorable à l'obtention des valeurs de H et Th souhaitées

Le pigment peut être non interférentiel et non fluorescent.

Dans le cas notamment d'un pigment dont les particules présentent une structure multicouche relativement transparente, la proportion de pigment peut être relativement élevée, étant par exemple supérieure ou égale à 1 % en poids, notamment comprise entre 1,5 et 10 % en poids, mieux entre 2 % et 8 % en poids, par rapport au poids total de la composition. La proportion peut par exemple être comprise entre 2 % et 5 %, étant par exemple de l'ordre de 3 %.

Plus la transparence du pigment est élevée, plus la quantité de pigment pourra, a priori, être importante sans faire perdre à la composition l'effet de flou et le facteur de transmission hémisphérique recherchés.

La transparence du pigment peut être quantifiée par son rapport de contraste, défini plus loin, celui-ci étant compris par exemple entre 15 et 65, mieux inférieur ou égal à 50. Le rapport de contraste peut notamment être inférieur ou égal à 45, mieux inférieur ou égal à 40, ou mieux encore 35.

L'agent de coloration peut comporter un pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

L'agent de coloration peut encore comporter un pigment ayant une structure qui peut être par exemple de type microsphères de silice contenant de l'oxyde de fer. Un exemple de pigment présentant cette structure et un rapport de contraste relativement faible est celui commercialisé par la société MIYOSHI sous la référence PC BALL PC-LL-100 P, ce pigment étant constitué de microsphères de silice contenant de l'oxyde de fer jaune.

Des valeurs relativement faibles de rapport de contraste, combinées aux valeurs d'indice de flou et de facteur de transmission hémisphérique, permettent d'obtenir des résultats particulièrement satisfaisants en termes d'apport conjoint de couleur et de couvrance, tout en gardant un effet de flou important, permettant de masquer les imperfections.

Dans le cas d'un mélange de pigments, les proportions de ceux-ci pourront être ajustées en fonction de leur rapport de contraste.

La composition peut être dépourvue de pigments de rapport de contraste supérieur à 60.

La composition peut encore, en variante, comporter des particules d'au moins un pigment qui est relativement opaque, notamment qui soit de rapport de contraste supérieur à 60, sous réserve que la quantité introduite dans la composition ne nuise pas à l'obtention des valeurs de H et Th recherchées.

La composition peut ainsi comporter, par exemple, des pigments conventionnels ayant un rapport de contraste relativement élevé, par exemple supérieur ou égal à 40, par exemple des pigments du type oxydes de fer et/ou dioxyde de titane.

La composition comporte de préférence moins de 2 % en poids de tels pigments, mieux moins de 1,5 %, mieux encore moins de 1 %, mieux encore moins de 0,75 %, par exemple entre 0,25 et 0,75 %, par exemple de l'ordre de 0,5 %, par rapport au poids total de la composition.

L'agent de coloration peut encore être choisi parmi les colorants, notamment les colorants hydrosolubles ou liposolubles ou d'autre matières colorantes encore, dans la mesure où leur incorporation à la composition ne nuit pas à l'obtention de l'indice de flou H et du facteur de transmission hémisphérique Th recherchés. L'agent de coloration peut ainsi comporter un colorant hydrosoluble tel que du caramel par exemple.

L'agent de coloration peut avoir une couleur correspondant à celle de la peau sur laquelle il est destiné à être appliqué, notamment comporter au moins un pigment brun, jaune ou rouge.

L'agent de coloration peut être dépourvu de nacres.

Lorsque la composition à effet de flou est pulvérisée sur une composition de base colorée, la composition à effet de flou peut contenir le cas échéant un agent de coloration quelconque dans la mesure où celui-ci n'affecte notamment pas les valeurs de H et Th recherchées.

Cet agent de coloration peut être choisi parmi toutes les matières colorantes conventionnellement utilisées.

### Mesure du rapport de contraste

Le rapport de contraste, encore appelé « contrast ratio » se trouve défini notamment dans la demande internationale WO 98/52534.

Pour calculer le rapport de contraste, on procède de la manière suivante.

Un mélange formé de 5 % en poids, par rapport au poids total, de l'agent de coloration à étudier et pour le reste de l'émulsion de référence ci-dessous est appliquée sur un support opaque noir et sur un support opaque blanc, selon un film d'une épaisseur de 50 µm. Ce film est séché pendant 24 heures à 25° C +/- 1 °C sous une pression de 1 atm.

L'émulsion de référence a pour formulation :

| | % en poids |
|---|---|
| - Eau | 45,83 |
| - p-hydroxybenzoate de méthyle | 0,45 |
| - Chlorphénesine | 0,34 |
| - Disodium EDTA | 0,11 |
| - Glycérine | 5,62 |
| - PEG-8 | 2,25 |
| - PEG-20 | 1,12 |
| - Silicate de magnésium et d'aluminium | 0,9 |
| - Lauroyl sarcosinate de sodium | 1,68 |
| - Dioxyde de titane (et) alumine (et) glycérine (et) silice | 3,37 |
| - Triéthanolamine | 1,35 |
| - Acide stéarique | 2,7 |
| - Stéarate de glycéryle | 2,02 |
| - p-hydroxybenzoate de butyle | 0,17 |
| - Isononanoate d'isononyle | 8,99 |
| - Cyclohexasiloxane | 6,57 |
| - Diméthicone | 10,28 |
| - BIS-PEG-15 méthyléther diméthicone | 2,25 |
| - Talc | 1,12 |
| - Kaolin | 1,12 |
| - Polyméthyl méthacrylate | 1,69 |

A l'aide d'un colorimètre, par exemple du type MINOLTA CR-200, en mode illuminant D65, angle de vue 0°, on mesure la valeur du tristimulus Y de la composition en trois endroits différents du support noir et en trois endroits différents du support blanc.

Le rapport de contraste correspond à la moyenne des trois valeurs de Y mesurées sur le support noir, divisée par la moyenne des trois valeurs de Y mesurées sur le support blanc, et multipliée par 100.

Plus le rapport de contraste est élevé et proche de 100 %, plus l'agent de coloration est opaque. Plus le rapport de contraste est faible, plus l'agent de coloration est transparent.

### Milieu physiologiquement acceptable

La composition à effet de flou comporte un milieu physiologiquement acceptable.

Par « milieu physiologiquement acceptable », on désigne un milieu non toxique et susceptible d'être appliqué sur la peau, notamment du visage et/ou du cou, les lèvres ou les ongles d'êtres humains.

Ce milieu physiologiquement acceptable peut comporter le cas échéant une phase grasse liquide, celle-ci pouvant comprendre au moins une huile choisie parmi les huiles volatiles, les huiles non volatiles et leurs mélanges.

### Huile volatile

La phase grasse liquide de la composition à effet de flou peut comprendre au moins une huile volatile.

Par « huile volatile », on entend tout milieu non aqueux susceptible de s'évaporer au contact de la peau en moins d'une heure, à température ambiante (25 °C) et pression atmosphérique (760 mm de Hg). La ou les huiles cosmétiques volatiles, liquides à température ambiante, ont notamment une pression de vapeur, mesurée à température ambiante et pression atmosphérique, allant de 10⁻³ à 300 mm de Hg (0,266 Pa à 40 000 Pa), de préférence de 0,02 mm à 300 mm de Hg (2,66 Pa à 40 000 Pa) et mieux allant de 0,1 à 90 mm de Hg (13 Pa à 12 000 Pa).

Ces huiles volatiles peuvent être des huiles hydrocarbonées, des huiles siliconées, comportant éventuellement des groupements alkyle ou alkoxy pendants ou en extrémité de chaîne siliconée, des huiles fluorées, ou un de leurs mélanges.

En particulier, les huiles volatiles peuvent être des huiles cosmétiques choisies parmi les huiles n'ayant pas de point éclair, les huiles ayant un point éclair allant de 40 °C à 100 °C, et leurs mélanges, en vue de faciliter leur mise en oeuvre. En outre, elles présentent avantageusement une température d'ébullition à pression atmosphérique inférieure à 220 °C et notamment inférieure à 210 °C, en particulier allant de 110 à 210 °C. Ces huiles volatiles peuvent par exemple ne pas être des monoalcools à au moins 7 atomes de carbone.

Comme huile volatile utilisable, on peut citer les huiles de silicones linéaires ou cycliques ayant une viscosité, à température ambiante, inférieure à 8 cSt et ayant notamment de 2 à 7 atomes de silicium, ces silicones comportant éventuellement des groupes alkyle ou alkoxy ayant de 1 à 10 atomes de carbone.

Comme huile de silicone volatile utilisable, on peut citer notamment l'octaméthylcyclo-tétrasiloxane, le décaméthylcyclopentasiloxane, le dodécaméthylcyclohexasiloxane, l'heptaméthylhexyltrisiloxane, l'heptaméthyloctyl trisiloxane, l'hexaméthyldisiloxane, l'octaméthyltrisiloxane, le décaméthyltétrasiloxane, le dodécaméthylpentasiloxane et leurs mélanges.

Comme autre huile volatile utilisable, on peut également citer les huiles volatiles hydrocarbonées ayant de 8 à 16 atomes de carbone et leurs mélanges et notamment les alcanes ramifiés en C₈-C₁₆ comme les isoalcanes (appelés aussi isoparaffines) en C₈-C₁₆, l'isododécane, l'isodécane, l'isohexadécane et par exemple les huiles vendues sous les noms commerciaux d'Isopars® ou de Permetyls®, les esters ramifiés en C₈-C₁₆ comme le néo pentanoate d'iso-hexyle et leurs mélanges.

En particulier, on utilise l'isododécane (Permetyls 99 A®), les isoparaffines en C₈-C₁₆ comme l'Isopar L, E, G ou H®, leurs mélanges, éventuellement associés au décaméthyl tétrasiloxane ou au cyclopentasiloxane, ou à leurs mélanges.

On peut également utiliser des huiles volatiles fluorées.

Les huiles volatiles peuvent être présentes dans la composition à effet de flou en une teneur allant par exemple de 5 à 97,5 % en poids, et notamment en une teneur allant de 20 à 75 % en poids par rapport au poids total de la composition.

### Huile non volatile

La composition à effet de flou peut comprendre également une fraction grasse non volatile. Cette fraction grasse non volatile peut comprendre au moins une huile non volatile.

Par « huile non volatile », on entend un corps gras liquide à température ambiante (20 °C) et qui ne s'évapore pas à cette même température.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines,
- les huiles de silicone phénylées, notamment les phényltriméthicones,
- les huiles d'origine végétale ou minérale, telles que l'huile de paraffine, de vaseline, le perhydrosqualène, la lanoline, l'huile d'abricot, l'huile de germes de blé, d'amande douce, de calophyllum, de sésame, de macadamia, de pépins de raisin, de colza, de coprah, d'arachide, de tournesol, de palme, de ricin, d'avocat, de jojoba, d'olive ou de germes de céréales, la fraction liquide du beurre de karité ; des esters d'acides gras et de polyol, en particulier les triglycérides liquides, le myristate d'isopropyle, l'huile de mygliol, le néopentanoate d'isostéaryle, le palmitate d'éthyl-2-hexyle, l'huile de ricin, l'acétyl-citrate de tri-butyle ; des alcools, en particulier l'octyl-2-dodecanol ; des acétylglycérides ; des octanoates, décanoates ou ricinoléates d'alcools ou de polyalcools ; des triglycérides d'acides gras ; des glycérides, les huiles fluorées et perfluorées ;
- les composés amidés, en particulier ceux décrits dans la demande PCT/FR98/01077 comme le N-néopentanoyl-2-octyl-docécylamine, le N-néopentanoyl-2-butyl-ocylamine, le N-(3,5,5-triméthyl-hexanoyl)-2-octyl-docécylamine, le N-(3,5,5-triméthyl-hexanoyl)-2-butyl-octylamine, et
- leurs mélanges.

En particulier, la composition à effet de flou peut comprendre une ou plusieurs huiles choisies parmi l'huile de sésame, la lanoline, le myristate d'isopropyle, l'huile de mygliol, le néopentanoate d'isostéaryle, le palmitate d'éthyl-2-hexyle, l'huile de ricin, l'acétyl-citrate de tri-butyle, l'octyl-2-dodécanol, le N-pentanoyl-2-octyl dodécylamine, les polydiméthylsiloxanes et/ou leurs mélanges.

La fraction grasse non volatile de la composition à effet de flou peut également comprendre une phase grasse non liquide et en particulier au moins un corps gras choisi parmi les cires, les gommes, les résines et/ou les corps gras pâteux d'origine animale, végétale, minérale ou synthétique, et leurs mélanges. La teneur en phase grasse non liquide dans la composition à effet de flou est ajustée pour que celle-ci puisse être appliquée par pulvérisation.

La fraction grasse non volatile de la composition à effet de flou peut être présente en une teneur allant de 1 à 85 % en poids, et notamment en une teneur allant de 1 à 30 % en poids, par rapport au poids total de la composition.

La composition à effet de flou peut également comporter une phase aqueuse. Cette phase aqueuse peut être constituée essentiellement d'eau. Toutefois, elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

Cette phase aqueuse (eau et éventuellement le solvant organique miscible à l'eau) peut être présente dans la composition à effet de flou en une teneur allant de 1 % à 95 % en poids, notamment allant de 3 % à 80 % en poids, et en particulier allant de 5 % à 60 %, en poids par rapport au poids total de la composition à effet de flou.

La composition à effet de flou peut également comporter un polymère filmogène tel que défini plus loin, ce polymère filmogène étant mis en oeuvre dans des conditions permettant la pulvérisation.

D'une manière générale, la composition à effet de flou peut contenir en outre les adjuvants habituels dans le domaine cosmétique, tels que les émulsionnants, les gélifiants hydrophiles ou lipophiles, les actifs hydrophiles ou lipophiles, les conservateurs, les antioxydants, les solvants, les parfums, les filtres solaires physiques et chimiques, les absorbeurs d'odeur et des ajusteurs de pH. Des exemples de tels adjuvants sont donnés plus loin.

La composition à effet de flou peut également contenir un tensioactif, notamment l'un de ceux mentionnés plus loin comme pouvant entrer dans la formulation de la composition de base.

La consistance, fluide, de la composition à effet de flou est susceptible de varier selon la nature du dispositif de pulvérisation utilisé et dans le cas particulier d'un dispositif de type aérosol, selon la nature de l'agent propulseur.

La composition à effet de flou se présentera avantageusement sous la forme d'une émulsion, obtenue par exemple par dispersion d'une phase grasse dans une phase aqueuse (H/E) ou inversement (E/H), d'une émulsion triple (E/H/E ou H/E/H), d'une dispersion vésiculaire de type ionique (liposomes ou oléosomes) et/ou non ionique (niosomes) et/ou d'une dispersion de nanocapsules ou nanosphères.

Dans le cas d'une émulsion, la proportion de la phase grasse de la composition considérée peut aller par exemple de 5 à 80 % en poids, et notamment de 5 à 50 % en poids par rapport au poids total de la composition.

La composition à effet de flou peut encore se présenter sous la forme d'un gel, capable d'être pulvérisé, notamment un gel thixotrope ou soluble dans le gaz propulseur par exemple.

### Composition de base

La composition de base, dans le cas où elle est appliquée, comporte également un milieu physiologiquement acceptable.

Ce milieu physiologiquement acceptable est généralement adapté à la nature du support sur lequel doit être appliquée la composition ainsi qu'à l'aspect sous lequel la composition est destinée à être conditionnée.

Ainsi, la composition de base peut être plus ou moins fluide et avoir l'aspect d'une crème colorée ou non, d'une pommade, d'un lait, d'une lotion, d'un sérum, d'une pâte, d'une mousse ou d'une poudre. Elle peut également se présenter sous forme solide, en particulier sous forme de stick ou de coupelle, ou avoir été déposée par enduction sur une feuille de papier.

La composition de base peut être sous forme anhydre, d'émulsion ou de gel.

La composition de base peut se présenter sous toutes formes galéniques normalement utilisées dans le domaine cosmétique, et elle peut être notamment sous forme d'une solution huileuse éventuellement gélifiée, d'une émulsion obtenue par dispersion d'une phase grasse dans une phase aqueuse (H/E) ou inversement (E/H), d'une émulsion triple (E/H/E ou H/E/H), d'un gel aqueux, d'une dispersion vésiculaire de type ionique (liposomes ou oléosomes) et/ou non ionique (niosomes) et/ou d'une dispersion de nanocapsules ou nanosphères.

Dans le cas d'une émulsion, la proportion de la phase grasse de la composition considérée peut aller par exemple de 5 à 80 % en poids, et notamment de 5 à 50 % en poids par rapport au poids total de la composition.

La composition de base peut comprendre au moins une phase aqueuse.

La phase aqueuse peut être constituée essentiellement d'eau. Toutefois, elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

Cette phase aqueuse (eau et éventuellement le solvant organique miscible à l'eau) peut être présente dans la composition de base en une teneur allant de 1 % à 95 % en poids, notamment allant de 3 % à 80 % en poids, et en particulier allant de 5 % à 60 %, en poids par rapport au poids total de la composition de base.

La composition de base peut également comprendre une phase grasse.

En particulier, la phase grasse de la composition de base peut comprendre au moins une huile, notamment choisie parmi :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; et
- leurs mélanges.

La composition de base peut également comprendre au moins une cire, au moins une gomme et/ou au moins un corps gras pâteux, d'origine végétale, animale, minérale ou de synthèse, siliconé ou non.

Les cires peuvent être hydrocarbonées, siliconées et/ou fluorées, comportant éventuellement des fonctions ester ou hydroxyle. Elles sont notamment d'origine naturelle.

La cire peut représenter de 0,01 à 10 % en poids, notamment de 0,1 à 5 % en poids, par rapport au poids total de la composition de base. Selon un mode de réalisation particulier, la composition de base peut être exempte de cires.

La composition de base peut comprendre en outre au moins un polymère filmogène.

Selon la présente invention, on entend par « polymère filmogène », un polymère apte à former à lui seul ou en présence d'un agent auxiliaire de filmification, un film continu et adhérent sur la peau.

On utilise avantageusement un polymère filmogène apte à former un film hydrophobe, c'est-à-dire un polymère dont le film a une solubilité dans l'eau à 25 °C inférieure à 1 % en poids.

Le polymère filmogène peut notamment être au moins un polymère choisi parmi le groupe comprenant :
- les polymères filmogènes hydrosolubles,
- les dispersions aqueuses de particules de polymères filmogènes hydrodispersibles, encore appelées « latex » ; dans ce cas, la composition de type fond de teint doit comprendre une phase aqueuse,
- les polymères filmogènes liposolubles,
- les polymères filmogènes lipodispersibles sous forme de dispersions non aqueuses de particules de polymère, de préférence des dispersions de particules polymériques, le cas échéant stabilisées en leur surface par au moins un agent stabilisant, dans une ou plusieurs huiles de silicones et/ou hydrocarbonées ; ces dispersions non aqueuses sont encore appelées « NAD », et
- leurs mélanges.

Comme dispersions aqueuses de polymère filmogène utilisables selon l'invention, on peut citer les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou bien encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère filmogène, on peut également citer les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Comme exemples de polymères filmogènes hydrosolubles utilisables selon l'invention, on peut citer les protéines comme les protéines d'origine végétale, les polymères de chitine ou de chitosane anioniques, cationiques, amphotères ou non-ioniques, les polymères de cellulose, les polymères ou copolymères acryliques, les polymères vinyliques, les polymères d'origine naturelle, éventuellement modifiés, et leurs mélanges.

Des dispersions de polymère filmogène dans la phase grasse liquide, en présence d'agent stabilisants, sont notamment décrites dans les documents EP-A-749746, EP-A-923928 et EP-A-930060.

A titre d'exemple de polymères liposolubles, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Le polymère filmogène peut être présent dans la composition en une teneur en matières sèches allant de 0,01 à 20 % en poids et notamment de 0,5 % à 10 % en poids par rapport au poids total de la composition.

La composition de base peut contenir également les adjuvants habituels dans le domaine cosmétique, tels que les émulsionnants, les gélifiants hydrophiles ou lipophiles, les matières colorantes, les actifs hydrophiles ou lipophiles, les conservateurs, les antioxydants, les solvants, les parfums, les charges, les filtres solaires physiques et chimiques, les absorbeurs d'odeur et des ajusteurs de pH.

Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse ou dans la phase aqueuse.

En tout état de cause, ces adjuvants, ainsi que leurs proportions, seront choisis de manière à ne pas nuire aux propriétés recherchées selon l'invention.

La composition de base peut comprendre en outre au moins un tensioactif.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions des tensioactifs, en particulier p.347-377 de cette référence, pour les tensioactifs anioniques et non-ioniques.

Parmi les tensioactifs pouvant être utilisés notamment dans la composition de base notamment on peut citer :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.
   Comme émulsionnants et co-émulsionnants utilisables, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H disponibles par exemple sous les dénominations commerciales ABIL WE09, ABIL EM 90 et ABIL EM97 auprès de la société Degussa Goldschmidt ou le mélange de stéarate d'éthylène glycol acétyle et de tristéarate de glycéryle commercialisé par la société Guardian sous la dénomination commerciale UNITWIX.
   Comme gélifiants hydrophiles utilisables, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.
   Comme matière colorante pouvant être utilisée notamment dans la composition de base, on peut citer les colorants lipophiles, les colorants hydrophiles, les pigments et les nacres habituellement utilisés dans les compositions cosmétiques ou dermatologiques, et leurs mélanges.
   Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.
   Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium.
   Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
   Les pigments peuvent avoir subi un traitement de surface.
   Parmi les pigments utilisables dans la composition de base, on peut encore citer les pigments goniochromatiques.
   Comme charges utilisables dans la composition de base, on peut citer, outre les charges décrites précédemment en relation avec la composition à effet de flou, les pigments, l'amidon réticulé par l'anhydride octénylsuccinique commercialisé par la société National Starch sous la dénomination DRY FLO PLUS (28-1160) ; les particules de polyamide et notamment celles vendues sous la dénomination ORGASOL par la société Atochem ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de types polyméthacrylate (PMMA), les élastomères de silicone et les poudres de silice de type SUNSPHERES, les microbilles de résine de silicone telles que celles commercialisées sous la dénomination TOSPEARL par la société Toshiba Silicone ; et leurs mélanges.
   Ces charges peuvent être présentes dans des quantités allant de 0,01 à 20 % en poids et de préférence de 1 à 10 % en poids par rapport au poids total de la composition de base.
   Des charges avantageuses pour une utilisation dans la présente invention sont la silice, le mica et le dioxyde de titane.

### Actifs

Comme actifs habituels dans le domaine cosmétique ou dermatologique pouvant être utilisés dans la composition de base et/ou dans la composition à effet de flou, on peut citer en particulier tous les actifs connus pour leur activité sur le vieillissement de la peau comme les agents kératolytiques ou prodesquamants, par exemple les α-hydroxy-acides, les β-hydroxy-acides, les α-céto-acides, les β-ceto-acides, les rétinoïdes et leurs esters, le rétinal, l'acide rétinoïque et ses dérivés. On peut citer aussi les extraits de plantes veinotoniques tels que les extraits de petit houx et/ou de marron d'Inde ; les bases xanthiques telles que la caféine ; les vitamines, telles que par exemple les vitamines A, B3, PP, B5, E, K1 et/ou C et les dérivés de ces vitamines et notamment leurs esters ; les agents anti-radicaux libres ; les filtres solaires ; les agents hydratants comme les polyols ; les céramides ; la DHEA et ses dérivés ; le coenzyme Q10 ; les agents blanchissants et dépigmentants comme l'acide kojique, les extraits de scutellaire, de mûrier, de réglisse et/ou de camomille ; les dérivés de para-aminophénols, l'arbutine et leurs dérivés, et leurs mélanges.

Pour une utilisation dans le traitement cosmétique des peaux grasses ou mixtes, la composition de base et/ou la composition à effet de flou peuvent contenir au moins un actif choisi parmi : les vitamines B3 et B5 ; les sels de zinc, et en particulier l'oxyde de zinc et le gluconate de zinc ; l'acide salicylique et ses dérivés tels que l'acide n-octanoyl-5-salicylique ; le triclosan ; la capryloylglycine ; un extrait de clou de girofle ; l'octopirox ; l'hexamidine ; et l'acide azélaïque et ses dérivés.

On peut également introduire dans la composition de base et/ou dans la composition à effet de flou des filtres UVA et/ou UVB, choisis parmi les filtres organiques et les filtres minéraux éventuellement enrobés pour les rendre hydrophobes.

### Kit de maquillage

La présente invention concerne également, selon un autre de ses aspects, un kit de maquillage. Ce kit de maquillage comprend au moins une composition de base et une composition à effet de flou selon l'invention, différente de la composition de base.

Ce kit de maquillage peut comprendre en outre des moyens d'application de la composition de base sur la peau par exemple.

La composition de base et la composition à effet de flou, formant ce kit de maquillage, peuvent être conditionnées dans des articles de conditionnement distincts ou non.

Les exemples de formulation figurant ci-après sont présentés à titre illustratif et non limitatif de l'invention, les pourcentages étant exprimés en poids par rapport au poids total de la composition.

### Exemple d'une composition de base constituée par un fond de teint

| | % en poids |
|---|---|
| - Butylène-1,3 glycol | 10,00 |
| - Hectorite modifiée par chlorure de di-stéaryl di-méthyl ammonium (commercialisée sous le nom BENTONE 38 V par ELEMENTIS) | 1,60 |
| - Conservateurs | 0,90 |
| - Cyclopenta diméthylsiloxane | 11,36 |
| - Néopentanoate d'iso-stéaryle | 0,50 |
| - Chlorure de sodium | 0,70 |
| - Isododécane | 13,00 |
| - Cyclohexadiméthylsiloxane | 8,00 |
| - Poly diméthylsiloxane (DC 200 Fluid 5 cst commercialisé par DOW CORNING) | 2,00 |
| - Cétyl diméthicone copolyol (commercialisé sous la dénomination ABIL EM90 par GOLDSCHMIDT) | 0,80 |
| - Isostéarate polyglycérol | 0,60 |
| - Isoeicosane | 2,00 |
| - Laurate d'hexyle | 0,60 |
| - Microsphères creuses de polyméthacrylate de méthyle (commercialisées sous la dénomination COVABEAD LH85 par WACKHERR) | 2,00 |
| - Poudre de polyméthylméthacrylate (commercialisée sous la dénomination JURYMER MB 1 par NIHON JUNYAKU) | 2,00 |
| - Poly diméthylsiloxane oxyéthyléné (commercialisé sous la dénomination KF-6017 de SHIN ETSU) | 4,48 |
| - Nacre | 1,00 |
| - Oxyde de fer jaune enrobé de phosphate de perfluoroalkyle en dispersion dans décaméthyl cyclopentasiloxane/diméthicone copolyol (commercialisé sous la dénomination FA50DYF par KOBO) | 2,40 |
| - Oxyde de fer brun enrobé de phosphate de perfluoroalkyle en dispersion dans cyclométhicone/diméthyl polysiloxane copolyol (commercialisé sous la dénomination FA50DRF par KOBO) | 0,93 |
| - Oxyde de fer noir enrobé de phosphate de perfluoroalkyle en dispersion dans décaméthyl cyclopentasiloxane/diméthicone copolyol (commercialisé sous la dénomination FA65DBF par KOBO) | 0,44 |
| - Oxyde de titane traité alumine enrobé de phosphate de perfluoroalkyle dans décaméthyl cyclopentasiloxane/- diméthicone copolyol (commercialisé sous la dénomination FA65DF par KOBO) | 9,54 |
| - Eau qsp | 100,00 |

### Exemple d'une composition non colorée à effet de flou destinée à être pulvérisée sur la composition de base ci-dessus :

| | % en poids |
|---|---|
| - Polyglycéryl-4 isostéarate (et) laurate d'hexyle (et) cétyl PEG/PPG-10/1 diméthicone | 9 |
| - Stéarate de glycol acétylé | 0,7 |
| - p-hydroxybenzoate de butyle | 0,15 |
| - 2-Oléamido-1,3-octadécanediol | 0,04 |
| - Cyclopentasiloxane (et) distéardimonium hectorite (et) alcool dénat. | 8 |
| - Cyclopentasiloxane | 14,25 |
| - Diméthicone | 4 |
| - Isododécane | 2,6 |
| - Talc P3 de la société NIPPON TALC | 8 |
| - Eau | 47,06 |
| - Sulfate de magnésium | 0,7 |
| - p-hydroxybenzoate de méthyle | 0,25 |
| - Propylène glycol | 5 |
| - Chlorphénesine | 0,25 |

La composition à effet de flou est appliquée par un dispositif de pulvérisation de type aérographe sur la composition de base.

On peut observer une diminution sensible de la perception des imperfections de la peau.

### Exemple d'une composition colorée à effet de flou pouvant être pulvérisée sur peau nue

| | % en poids |
|---|---|
| - Polyglycéryl-4 isostéarate (et) laurate d'hexyle (et) cétyl PEG/PPG- 10/1 diméthicone | 9 |
| - Stéarate de glycol acétylé | 0,7 |
| - p-hydroxybenzoate de butyle | 0,15 |
| - 2-oléamido-1,3-octadécanediol | 0,04 |
| - Cyclopentasiloxane (et) distéardimonium hectorite (et) alcool dénat. | 8 |
| - Cyclopentasiloxane | 14,25 |
| - Diméthicone | 4 |
| - Isododécane | 2,6 |
| - Pigment COVERLEAF NS de la société CHEMICALS AND CATALYSTS | 3 |
| - Talc P3 de la société NIPPON TALC | 8 |
| - Eau | 44,06 |
| - Sulfate de magnésium | 0,7 |
| - p-hydroxybenzoate de méthyle | 0,25 |
| - Propylène glycol | 5 |
| - Chlorphénesine | 0,25 |
| | |

La composition est appliquée avec un dispositif de pulvérisation de type aérographe.

On observer un apport de couleur et une diminution de la perception des défauts de reliefs.

### Exemple d'une autre composition colorée à effet de flou pouvant être pulvérisée sur peau nue

| | % en poids |
|---|---|
| - Polyglycéryl-4 isostéarate (et) laurate d'hexyle (et) cétyl PEG/PPG-10/1 diméthicone | 9 |
| - Stéarate de glycol acétylé | 0,7 |
| - p-hydroxybenzoate de butyle | 0,15 |
| - 2-Oléamido-1,3-octadécanediol | 0,04 |
| - Cyclopentasiloxane (et) distéardimonium hectorite (et) alcool dénat. | 8 |
| - Cyclopentasiloxane | 14,25 |
| - Diméthicone | 4 |
| - Isododécane | 2,6 |
| - Oxydes de fer (et) glutamate de stéaroyle disodium (et) hydroxyde d'aluminium | 0,18 |
| - Dioxyde de titane (et) glutamate de stéaroyle disodium (et) hydroxyde d'aluminium | 0,32 |
| - Talc P3 de la société NIPPON TALC | 8 |
| - Eau | 46,56 |
| - Sulfate de magnésium | 0,7 |
| - p-hydroxybenzoate de méthyle | 0,25 |
| - Propylène glycol | 5 |
| - Chlorphénesine | 0,25 |

La composition est appliquée comme la précédente au moyen d'un dispositif de pulvérisation de type aérographe. On observe également une diminution de la visibilité des imperfections de couleur et de relief de la peau.

Dans toute la description, y compris les revendications, l'expression « comportant un » doit être comprise comme étant synonyme de « comportant au moins un », sauf si le contraire est spécifié.

## Revendications

1. Procédé de traitement cosmétique, notamment de maquillage, d'une surface de la peau, des muqueuses ou des ongles, comportant :
- l'application sur ladite surface par pulvérisation d'une composition cosmétique à effet flou, cette composition comportant, dans un milieu physiologiquement acceptable, au moins une charge constituée de particules ayant une taille moyenne en nombre inférieure à 5 µm, et permettant de conférer à la composition un facteur de transmission hémisphérique Th supérieur ou égal à 70 % et un indice de flou H supérieur ou égal à 40 % avec H désignant la grandeur ((Th-Td)/Th) 100 dans laquelle Th représente le facteur de transmission hémisphérique et Td le facteur de transmission directe.

2. Procédé selon la revendication 1, **caractérisée en ce que** la composition présente un indice de flou supérieur ou égal à 45 %, notamment supérieur ou égal à 50 %, en particulier supérieur ou égal à 60 %, voire supérieur ou égal à 70 %, mieux à 80 %.

3. Procédé selon la revendication 1 ou 2, **caractérisée en ce que** la composition présente un facteur de transmission hémisphérique Th supérieur ou égal à 75 %, notamment supérieur ou égal à 80 % et en particulier supérieur ou égal à 85 %.

4. Procédé selon l'une quelconque des revendications précédentes, **caractérisé en ce que** la charge est choisie parmi les poudres de silice ou de silicates notamment d'alumine, le talc, les poudres de type polyméthylméthacrylate (PMMA), les composés silice/TiO₂, ou silice/oxyde de zinc, les poudres de polyéthylène, les poudres d'amidon, les poudres de polyamides, les poudres de copolymères styrène/acrylique, les élastomères de silicone, et leurs mélanges.

5. Procédé selon l'une quelconque des revendications précédentes, **caractérisé en ce que** la charge est choisie parmi les particules de talc de taille moyenne inférieure ou égale à 3 µm, la poudre de Nylon^{®} 12, les particules de silice traitées en surface par une cire minérale, les microsphères de silice amorphe, les microbilles de silice, et leurs mélanges.

6. Procédé selon l'une quelconque des revendications précédentes, **caractérisé en ce que** la charge est présente à raison de 0,1 à 20 % en poids et notamment de 1 à 12 %, mieux de 5 à 10 % en poids, notamment environ 8 % en poids par rapport au poids total de la composition.

7. Procédé selon l'une quelconque des revendications précédentes, **caractérisée en ce que** la composition comprend au moins une phase grasse liquide, comprenant au moins une huile choisie parmi les huiles volatiles, les huiles non volatiles, et leurs mélanges.

8. Procédé selon la revendication 7, **caractérisé en ce que** la composition comprend au moins une huile volatile choisie parmi les huiles de silicone linéaires ou cycliques ayant une viscosité à température ambiante inférieure à 8 cSt, les huiles volatiles hydrocarbonées ayant de 8 à 16 atomes de carbone, les huiles fluorées et leurs mélanges.

9. Procédé selon la revendication 7 ou 8, **caractérisé en ce que** la composition comprend au moins une huile volatile choisie parmi l'iso-dodécane, les iso-paraffines en C₈ à C₁₆, et leurs mélanges.

10. Procédé selon la revendication 9, **caractérisé en ce que** la composition comprend au moins une huile volatile associée avec du décaméthyltétrasiloxane, du cyclopentasiloxane, ou un mélange de ceux-ci.

11. Procédé selon l'une quelconque des revendications 7 à 10, **caractérisé en ce que** la composition comprend au moins une huile volatile en une teneur allant de 5 à 97,5 % en poids et notamment en une teneur allant de 20 à 75 % en poids, par rapport au poids total de la composition.

12. Procédé selon l'une quelconque des revendications précédentes, **caractérisé en ce que** la phase grasse liquide comprend au moins une huile non volatile, choisie parmi les polyalkyles (C₁ - C₂₀) siloxanes, les silicones modifiés par des groupements aliphatiques et/ou aromatiques, les huiles de silicone phénylées, les huiles d'origine végétale ou minérale, les composés amidés et leurs mélanges.

13. Procédé selon la revendication 12, **caractérisé en ce que** l'huile non volatile est choisie parmi l'huile de sésame, la lanoline, le myristate d'isopropyle, l'huile de mygliol, le néopentanoate d'isostéaryle, le palmitate d'éthyl-2-hexyle, l'huile de ricin, l'acétyl-citrate de tri-butyle, l'octyl-2-dodecanol, le N-pentanoyl-2-octyl dodécylamine, les polydiméthylsiloxanes, et leurs mélanges.

14. Procédé selon l'une quelconque des revendications précédentes **caractérisé en ce que** la composition comprend en outre une phase grasse non liquide.

15. Procédé selon l'une quelconque des revendications précédentes, **caractérisé en ce que** la composition comprend une fraction grasse non volatile présente à raison de 1 à 85 % en poids, et notamment de 1 à 30 % en poids par rapport au poids total de la composition.

16. Procédé selon l'une quelconque des revendications précédentes **caractérisé en ce qu'**il comprend en outre l'application préalable sur la surface d'au moins une composition de base différente de la composition à effet de flou avant l'étape de pulvérisation de la composition à effet de flou.

17. Procédé de la revendication 16, **caractérisé par le fait que** la composition de base est colorée.

18. Procédé selon l'une quelconque des revendications 1 à 17, **caractérisé en ce que** la pulvérisation de la composition dite à effet de flou est effectuée simultanément à la pulvérisation d'au moins une composition de base distincte de ladite composition à effet de flou.

19. Procédé selon l'une quelconque des revendications 16 à 18, **caractérisé en ce que** l'une au moins de la composition à effet de flou et de la composition de base comporte au moins un agent de coloration.

20. Kit de maquillage comprenant au moins une composition cosmétique de base et une composition à effet de flou à appliquer par pulvérisation différente de la composition de base, telle que définie dans l'une quelconque des revendications 1 à 19.

21. Dispositif de pulvérisation comportant un récipient contenant une composition comportant dans un milieu physiologiquement acceptable, au moins une charge constituée de particules ayant une taille moyenne en nombre inférieure à 5 µm, et dispositif permettant de conférer à la composition un facteur de transmission hémisphérique Th supérieur ou égal à 70 %, un indice de flou H supérieur ou égal à 40 % avec H désignant la grandeur ((Th-Td)/Th) 100 dans laquelle Th représente le facteur de transmission hémisphérique et Td le facteur de transmission directe.

22. Dispositif selon la revendication 21, **caractérisé par le fait qu'**il comporte des moyens pour soumettre la composition à une pression permettant de la pulvériser.

23. Dispositif selon la revendication 21, **caractérisé par le fait qu'**il comporte des moyens pour soumettre la composition à une dépression permettant de la pulvériser.

## Claims

1. Cosmetic treatment process, especially a makeup process, for a surface of skin, mucous membranes or the nails, comprising:
- the application to the said surface, by spraying, of a cosmetic soft-focus composition, this composition comprising, in a physiologically acceptable medium, at least one filler consisting of particles with a number-average size of less than 5 µm and that gives the composition a hemispheric transmission factor Th of greater than or equal to 70% and a haze index H of greater than or equal to 40% with H denoting the magnitude ((Th-Td)/Th), 100, in wich Th represents the hemispheric transmission factor and Td represents the direct transmission factor.

2. Process according to claim 1, wherein the composition has a haze index H of greater than or equal to 45%, especially greater than or equal to 50%, especially greater than or equal to 60%, even better greater than or equal to 70%, and better still greater than or equal to 80%.

3. Process according to claim 1 or 2, wherein the composition has a hemispheric transmission factor Th of greater than or equal to 75%, especially greater than or equal to 80% , even better greater than or equal to 85%.

4. Process according to anyone of the preceding claims, wherein the filler is chosen from the group consisting of silica or silicate powders in particular alumina silicate powders, talc, powders of polymethyl methacrylate (PMMA) type, silica/TiO₂ or silica/zinc oxide composites, polyethylene powders, starch powders, polyamide powders, styrene/acrylic copolymer powders and silicone elastomers, and mixtures thereof.

5. Process according to anyone of the preceding claims, wherein the filler is chosen from the group consisting of talc particles with an average size of less than or equal to 3 µm, Nylon^{®} 12 powder, silica particles surface-treated with a mineral wax, amorphous silica microspheres and silica microbeads, and mixtures thereof.

6. Process according to anyone of the preceding claims, wherein the filler is present in a proportion of from 0.1% to 20% by weight, in particular from 1% to 12%, even better from 5% to 10%, especially about 8% by weight, relative to the total weight of the composition.

7. Process according to anyone of the preceding claims, wherein the composition comprises at least one liquid fatty phase, comprising at least one oil chosen from the group consisting of volatile oils and non-volatile oils, and mixtures thereof.

8. Process according to claim 7, wherein the composition comprises at least one volatile oil chosen from the group consisting of linear or cyclic silicone oils with a viscosity at room temperature of less than 8 cSt, volatile hydrocarbon-based oils containing from 8 to 16 carbon atoms and fluoro oils, and mixtures thereof.

9. Process according to claim 7 or 8, wherein the composition comprises at least one volatile oil chosen from the group consisting of isododecane and C₈ to C₁₆ isoparaffins, and mixtures thereof.

10. Process according to claim 9, wherein the composition comprises at least one volatile oil combined with decamethyltetrasiloxane or cyclopentasiloxane, or a mixture thereof.

11. Process according to anyone of claims 7 to 10, wherein the composition comprises at least one volatile oil in a content ranging from 5% to 97.5% by weight, in particular from 20% to 75% by weight, relative to the total weight of the composition.

12. Process according to anyone of the preceding claims, wherein the liquid fatty phase comprises at least one non-volatile oil chosen from the group consisting of poly(C₁-C₂₀)alkylsiloxanes, silicones modified with aliphatic and/or aromatic groups, phenylsilicone oils, oils of plant or mineral origin and amide compounds, and mixtures thereof.

13. Process according to claim 12, wherein the non-volatile oil is chosen from the group consisting of sesame oil, lanolin, isopropyl myristate, Miglyol oil, isostearyl neopentanoate, 2-ethylhexyl palmitate, castor oil, acetyl tributyl citrate, 2-octyldodecanol, N-pentanoyl-2-octyldodecylamine and polydimethylsiloxanes, and mixtures thereof.

14. Process according to anyone of the preceding claims, wherein the composition also comprises a non-liquid fatty phase.

15. Process according to anyone of the preceding claims, wherein the composition comprises a non-volatile fatty fraction present in a proportion of from 1% to 85% by weight, especially from 1% to 30% by weight, relative to the total weight of the composition.

16. Process according to anyone of the preceding claims, also comprising the prior application to the surface of at least one base composition that is different from the soft-focus composition, before the step of spraying the soft-focus composition.

17. Process according to claim 16, wherein the base composition is coloured.

18. Process according to anyone of claims 1 to 17,wherein the spraying of the soft-focus composition is performed simultaneously with the spraying of at least one base composition that is different from the said soft-focus composition.

19. Process according to anyone of claims 16 to 18, wherein at least one from among the soft-focus composition and the base composition comprises at least one colouring agent.

20. Makeup kit comprising at least one cosmetic base composition and a soft-focus composition to be applied by spraying that is different from the base composition, as defined in anyone of claims 1 to 19.

21. Spraying device comprising a container containing a composition comprising, in a physiologically acceptable medium, at least one filler consisting of particles with a number-average size of less than 5 µm, and that gives the composition a hemispheric transmission factor Th of greater than or equal to 70% and a haze index H of greater than or equal to 40% with H denoting the magnitude ((Th-Td)/Th).100, in wich Th represents the hemispheric transmission factor and Td represents the direct transmission factor.

22. Device according to claim 21, comprising means for subjecting the composition to a pressure that allows it to be sprayed.

23. Device according to claim 21, comprising means for subjecting the composition to a negative pressure that allows it to be sprayed.

## Patentansprüche

1. Verfahren zur kosmetischen Behandlung, insbesondere zum Schminken, von einer Hautoberfläche von Schleimhäuten oder von Nägeln bzw. Fingernägeln, welches aufweist:
- die Applikation auf der besagten Oberfläche durch Pulverisieren von einer kosmetischen Zusammensetzung mit weich- bzw. zartmachender Wirkung, wobei die Zusammensetzung in einer physiologisch annehmbaren Umgebung aufweist, zumindest einen Bestandteil, der aus Teilchen gebildet ist, die eine in der Anzahl mittlere Größe kleiner als 5 µm haben, und es ermöglichen, der Zusammensetzung einen halbkugelförmigen Transmissionsfaktor Th größer als oder gleich 70 % und eine Unschärfekennzahl H größer als oder gleich 40 % zu verleihen, wobei H die Größe ((Th-Td)/Th) 100 benennt, in welcher Th den halbkugelförmigen Transmissionsfaktor und Td den direkten Transmissionsfaktor darstellt.

2. Verfahren nach Anspruch 1, **dadurch gekennzeichnet, dass** die Zusammensetzung eine Unschärfe- bzw. Vergrößerungskennzahl größer als oder gleich 45 %, insbesondere größer als oder gleich 50 %, und insbesondere größer als oder gleich 60 %, gerne größer als oder gleich 70 %, besser 80 % vorzuweisen hat.

3. Verfahren nach Anspruch 1 oder 2, **dadurch gekennzeichnet, dass** die Zusammensetzung einen halbkugelförmigen Transmissionsfaktor Th größer als oder gleich 75 %, insbesondere größer als oder gleich 80 % und bevorzugt größer als oder gleich 85 % vorzuweisen hat.

4. Verfahren nach irgendeinem der voranstehenden Ansprüche, **dadurch gekennzeichnet, dass** der Bestandteil unter den Pulvern des Siliziums oder der Silikate, insbesondere von Aluminium, dem Talkum, den Pulvern vom Typ des Polymethylmetacrylat (PMMA), den Zusammensetzungen der Kieselerde/TiO₂, oder der Kieselerde/Oxids von Zink, den Pulvern des Polyethylens, den Pulvern des Stärkemehls, den Pulvern der Polyamide, den Pulvern der Styrol-/Acrylat-Copolymere, den Siliziumelastomeren und deren Mischungen ausgewählt ist.

5. Verfahren nach irgendeinem der voranstehenden Ansprüche, **dadurch gekennzeichnet, dass** der Bestandteil unter den Teilchen des Talkums mit einer durchschnittlichen Größe kleiner als oder gleich 3 µm, den Pulvern aus Nylon^{®} 12, den Teilchen der Kieselerde mit einer Oberflächenbehandlung über ein gereinigtes Erdwachs (Ceresin), den Mikrokugeln aus amorpher Kieselerde, den Glaskügelchen aus Kieselerde und deren Mischungen ausgewählt ist.

6. Verfahren nach irgendeinem der voranstehenden Ansprüche, **dadurch gekennzeichnet, dass** der Bestandteil in einem Verhältnis von 0,1 bis 20 Gew.-% und insbesondere von 1 bis 12 %, besser 5 bis 10 Gew.-%, bevorzugt ungefähr 8 Gew.-%, in Bezug auf das Gesamtgewicht der Zusammensetzung vorhanden ist.

7. Verfahren nach irgendeinem der voranstehenden Ansprüche, **dadurch gekennzeichnet, dass** die Zusammensetzung zumindest eine fettige Flüssigphase aufweist, die zumindest ein Öl aufweist, das unter den flüchtigen Ölen, den nicht flüchtigen Ölen und deren Mischungen ausgewählt ist.

8. Verfahren nach Anspruch 7, **dadurch gekennzeichnet, dass** die Zusammensetzung zumindest ein flüchtiges Öl aufweist, das unter den linearen Silikonölen oder den zyklischen Silikonölen, die eine Viskosität bei einer Umgebungstemperatur unterhalb von 8 cSt haben, wobei die flüchtigen teerhaltigen bzw. bituminösen Öle von 8 bis 16 Kohlenstoffatomen haben, den fluorierten Ölen und deren Mischungen ausgewählt ist.

9. Verfahren nach Anspruch 7 oder 8, **dadurch gekennzeichnet, dass** die Zusammensetzung zumindest ein flüchtiges Öl aufweist, das unter den Iso-dodecanen, Isoparafinen von C₈ bis C₁₆, und deren Mischungen ausgewählt ist.

10. Verfahren nach Anspruch 9, **dadurch gekennzeichnet, dass** die Zusammensetzung zumindest ein flüchtiges Öl aufweist, das mit Dekamethyltetrasiloxan, Zyklopentasiloxan oder einer Mischung von diesen vereinigt bzw. vermengt ist.

11. Verfahren nach irgendeinem der Ansprüche 7 bis 10, **dadurch gekennzeichnet, dass** die Zusammensetzung zumindest ein flüchtiges Öl mit einem Anteil von 5 bis 97, 5 Gew.-% und insbesondere einem Gehalt von 20 bis 75 Gew.-% in Bezug auf das Gesamtgewicht der Zusammensetzung aufweist.

12. Verfahren nach irgendeinem der voranstehenden Ansprüche, **dadurch gekennzeichnet, dass** die Fettflüssigphase zumindest ein nicht flüchtiges Öl aufweist, das unter den Polyalkyl (C₁-C₂₀)-Siloxanen, den Silikonen, die durch aliphatische und/oder aromatische Gruppen modifiziert sind, den phenylhaltigen Silikonölen, den Ölen mit vegetativem oder mineralischem Ursprung, den zusammengesetzten Amiden bzw. Fettsäureamiden und deren Mischungen ausgewählt ist.

13. Verfahren nach Anspruch 12, **dadurch gekennzeichnet, dass** das nichtflüchtige Öl ausgewählt ist unter dem Sesamöl, dem Lanolin, dem Isopropylmyristat, Mygliolöl, Isostearyl Neopentanoat, der Ethyl-2-Hexylpalmitinsäure, Rizinusöl, Tri-butyl-Acetylcitrat, Octyl-2-Dodencanol, N-pentanoyl-2-Octyl Dodecylamin, Polydimenthylsiloxanen und deren Mischungen.

14. Verfahren nach irgendeinem der voranstehenden Ansprüche, **dadurch gekennzeichnet, dass** die Zusammensetzung zusätzlich eine nichtflüssige Fettphase aufweist.

15. Verfahren nach irgendeinem der voranstehenden Ansprüche, **dadurch gekennzeichnet, dass** die Zusammensetzung eine nichtflüchtigen Fettanteil aufweist, der einen Anteil von 1 bis 85 Gew.-%, und insbesondere von 1 bis 30 Gew.-% in Bezug auf das Gesamtgewicht der Zusammensetzung repräsentiert.

16. Verfahren nach irgendeinem der voranstehenden Ansprüche, **dadurch gekennzeichnet, dass** dieses ferner die vorangehende Applikation auf der Oberfläche von zumindest einer Zusammensetzung mit unterschiedlicher Grundlage zu der Zusammensetzung mit der Wirkung der Vergrößerung bzw. Unschärfe vor dem Abschnitt der Pulverisierung von der Zusammensetzung mit der Wirkung der Vergrößerung bzw. Unschärfe aufweist.

17. Verfahren nach Anspruch 16, **dadurch gekennzeichnet, dass** die Grundzusammensetzung gefärbt ist.

18. Verfahren nach irgendeinem der Ansprüche 1 bis 17, **dadurch gekennzeichnet, dass** die Pulverisierung der Zusammensetzung, der der Vergrößerungs- bzw. Unschärfeeffekt zuzuschreiben ist, gleichzeitig mit der Pulverisierung von zumindest einer Grundzusammensetzung bewirkt wird, die sich von der besagten Zusammensetzung mit der Wirkung der Vergrößerung bzw. Unschärfe unterscheidet.

19. Verfahren nach irgendeinem der Ansprüche 16 bis 18, **dadurch gekennzeichnet, dass** zumindest eine von der Zusammensetzung mit der Wirkung der Vergrößerung bzw. Unschärfe und der Grundzusammensetzung zumindest ein Färbungsmittel aufweist.

20. Kit zum Schminken, das zumindest eine kosmetische Grundzusammensetzung und eine Zusammensetzung mit der Wirkung der Vergrößerung bzw. Verunschärfung, der von der Grundzusammensetzung abweicht, zum Applizieren durch Pulverisieren aufweist, von der Art, wie sie in irgendeinem der Ansprüche 1 bis 19 definiert ist.

21. Vorrichtung zum Pulverisieren, die einen Behälter aufweist, der eine Zusammensetzung enthält, die in einer annehmbaren physiologischen Umgebung zumindest einen Bestandteil aufweist, der aus Teilchen gebildet ist, die eine durchschnittliche Größe kleiner als 5 µm haben ist, und es ermöglicht, der Zusammensetzung einen hemisphärischen bzw. halbkugelförmigen Transmissionsfaktor Th größer als oder gleich 70 %, eine Kennzahl der Vergrößerung bzw. Unschärfe H größer als oder gleich 40 % zu verleihen, wobei H die Größe ((Th-Td)/Th) 100 zuordnet, bei welcher Th den halbkugelförmigen Transmissionsfaktor und Td den direkten bzw. unmittelbaren Transmissionsfaktor darstellt.

22. Vorrichtung nach Anspruch 21, **dadurch gekennzeichnet, dass** diese Mittel aufweist, um die Zusammensetzung einem Druck zu unterwerfen, der die Pulverisation erlaubt.

23. Vorrichtung nach Anspruch 21, **dadurch gekennzeichnet, dass** diese Mittel aufweist, um die Zusammensetzung einem Unterdruck zu unterwerfen, der die Pulverisation ermöglicht.
